# EUROPEAN PATENT APPLICATION

(11) **EP 2 708 200 A2**
(43) Date of publication of application: **19.03.2014**
(21) Application number: 12782862.2
(22) Date of filing: 30.04.2012
(51) Int. Cl.: A61B 18/14, A61N 1/06, A61B 17/34, A61M 25/01

(54) **HIGH-FREQUENCY HEAT THERAPY ELECTRODE DEVICE EQUIPPED WITH FLEXIBLE TUBE**

(30) Priority: 12.05.2011 KR 20110044720
(71) Applicant: Taewoong Medical Co., Ltd., Gimpo-si, Gyeonggi-do 415-873 (KR); Shin, Kyung-Min, Seoul 120-090 (KR)
(72) Inventor: SHIN, Kyung Min, Seoul 120-090 (KR); SHIN, Kyung Hoon, Gimpo-si Gyeonggi-do 415-738 (KR); KIM, Dong Un, Gimpo-si Gyeonggi-do 415-781 (KR); SEO, Dong Wan, Seoul 138-931 (KR)
(74) Representative: Klinski, Robert
(86) International application number: PCT/KR2012/003361
(87) International publication number: WO 2012/153928

(57) **Abstract**

The present invention relates to a high-frequency heat therapy electrode device, and one embodiment of the present invention provides a high-frequency heat therapy electrode device provided with an electrode needle disposed in front of a handle, which necrotizes a lesion site by cauterizing the site by means of the high-frequency heat generated from the electrode needle. The high-frequency heat therapy electrode device equipped with a flexible tube is characterized in that: a flexible tube of a prescribed hardness, but that can be easily bent and deformed, is disposed between the handle and the electrode needle such that the needle can be inserted, along the working channel of an endoscope, up to a lesion site and the electrode needle is provided with a cooling line in the inside thereof.

## Description

### [Technical Field]

The present invention relates to a high-frequency heat therapy electrode device which heats a lesion site such as carcinomatous tissue of body organs with high-frequency to cauterize and necrotize the lesion site, more particularly, to a high-frequency heat therapy electrode device provided with a flexible tube which has sufficient hardness and is easily bent and deformed to allow the flexible tube to be inserted along a working channel of an endoscope.

### [Background Art]

In general, if carcinomatous tissue is formed on a body organ such as liver, a lesion site is treated by nonoperative methods or surgical operations.

At this time, the surgical operation is disadvantageous in that since a body corresponding to a lesion site should be excised, an operative region is extremely large so that a body is largely scarred, and a considerable time for convalescing is required.

In addition, there are possibilities of recurrence of carcinomatous tissue and the like, if carcinomatous tissue recurs, the reoperation should be carried out so that a patient has a physical pain as well as an economic burden and risks.

Thus, in recent, the nonoperative methods, for example, carotid chemoembolization, percutaneous ethanol injection therapy, systemic anticancer chemotherapy, local thermal therapy and the like have been employed. It has been known that, among the above, the local thermal therapy is most effective for improving a short-term treatment result or a long-term survival rate.

High-frequency heat therapy, microwave cauterization, laser cauterization and the like belong to the local thermal therapy, and among the above, the high-frequency heat therapy has been most effectively employed.

In the high-frequency heat therapy, if carcinomatous tissue is formed on a body organ, for example, liver, liver is not excised, but only carcinomatous tissue is cauterized by high-frequency heat and then necrotized.

In an electrode device for the above high-frequency heat therapy, an electrode needle is assembled at a front end of a handle on which the operator grips and an electrode line for supplying high-frequency is connected to the electrode needle.

And, the operator inserts the electrode needle in the lesion site such as carcinomatous tissue of human organ to allow the electro needle to penetrate the lesion site and then supplies the high-frequency from a high-frequency generating device to the electrode needle to cauterize and necrotize the lesion site with high-frequency heat.

In the field of medicine, meanwhile, an endoscope is equipment inserting a thin and long inserting element in a tubular organ such as coelom, to observe an organ such as large intestine. If necessary, various medical treatments are performed by means of a treatment tool to be inserted in a working channel of the endoscope.

At this time, in order to perform the high-frequency heat therapy utilizing the working channel of the endoscope, there is a need to insert the electrode needle up to the lesion site along the working channel. However, since an entire body of a conventional electrode needle is formed of metal material such as stainless steel, the electrode needle has an inferior flexibility so that it is difficult to insert the electrode needle in the working channel which is being bent according to a shape of tubular organ in the human body.

In addition, if a body of the electrode needle is formed of flexible material such as polymer to solve the above problem, the sufficient insertion force for moving the electrode needle to the lesion site and for sticking the electrode needle in the tissue is not transmitted to the electrode needle.

In addition, when the high-frequency heat therapy is performed, while the tissue is coagulated and necrotized by high-frequency frictional heat, moisture in the tissue is simultaneously vaporized by heat. As a result, a carbonization phenomenon in which the lesion site is stuck to an end of the electrode needle is generated.

The above carbonization of the electrode needle causes a difficulty of high-frequency flow of the electrode needle to make it difficult to cauterize the lesion site, and also causes a difficulty of a separation of the electrode needle so that there is need to maximally suppress moisture vaporization from the tissue caused by heat.

### [Disclosure]

### [Technical Problem]

The present invention is conceived to solve the above-mentioned problems, one embodiment of the present invention relates to a high-frequency heat therapy electrode device provided with an electrode needle disposed in front of a handle and cauterizing a lesion site with the high-frequency heat generated from the electrode needle to necrotize the lesion site, the device comprises a flexible tube provided between the handle and the electrode needle, having a prescribed hardness and being easily bent and deformed, the flexible tube can be inserted up to the lesion site along a working channel of an endoscope and a cooling line is provided in the electrode needle.

### [Technical Solution]

According to a preferred embodiment of the present invention, a high-frequency heat therapy electrode device provided with an electrode needle disposed in front of a handle and cauterizing a lesion site with the high-frequency heat generated from the electrode needle to necrotize the lesion site is provided, and the device comprises flexible tube provided between the handle and the electrode needle and being easily bent and deformed.

Here, it is preferable that the flexible tube has a prescribed hardness to enable the electrode needle to be inserted into the lesion site along a working channel of an endoscope by means of a movement of the handle.

Also, the electrode needle is accommodated in a sheath tube and a front end portion of the electrode needle is exposed out of the sheath tube when the device is used.

At this time, the sheath tube is provided with an electrode line received therein, and a front end of the electrode line is connected to one side of an outer circumference surface of the electrode needle.

In addition, the handle comprises a gripping part and a sliding part which can be slid into the gripping part, and the sheath tube can be moved together with the sliding part.

Meanwhile, a cooling water circulation block for supplying and circulating cooling water into the electrode needle is provided at one side of an interior of the handle.

At this time, the cooling water circulation block may include a first block to which a cooling water supplying tube and a cooling water discharging tube are connected, the first block having a cooling water supplying passage and a cooling water discharging passage formed therein; a second block to which the cooling water discharging passage is extended, the second block being coupled to one side of the first block and communicated with the cooling water supplying passage; and a third block coupled to one side of the second block and communicated with the cooling water discharging passage.

Also, a guide tube extended into the electrode needle is coupled to one side of the cooling water circulation block for supplying cooling water.

And, it is preferable to provide a temperature-measuring sensor at one side of an interior of the guide tube.

In the meantime, a pushing rod may be provided between the flexible tube and the handle.

### [Description of Drawings]

Fig. 1 is a perspective view of a high-frequency heat therapy electrode device equipped with a flexible tube according to one embodiment of the present invention;
Fig. 2 is a schematic view showing a state in which cooling water is supplied to an electrode needle according to one embodiment of the present invention;
Fig. 3 is a schematic view showing a state in which cooling water is collected from an electrode needle according to one embodiment of the present invention;
Fig. 4 is a view showing a state in which a high-frequency heat therapy electrode device equipped with a flexible tube according to one embodiment of the present invention is in use; and
Fig. 5 is a view showing a state in which a high-frequency heat therapy electrode device equipped with a flexible tube according to another embodiment of the present invention is in use.

### [Mode for Invention]

Hereinafter, a high-frequency heat therapy electrode device equipped with a flexible tube according to a preferred one embodiment of the present invention is described with reference to the accompanying drawings. For clarity and convenience for a description, a thickness of each line and a size of each structural element shown in the drawings may be exaggeratedly illustrated.

Also, the terms described later are defined in view of a function in the present invention, however, the terms may be changed according to an intension of a user and an operator or a custom. Thus, a definition of the terms should be defined on the basis of the content of the entire specification.

In addition, the below embodiment does not limit the claims of the present invention, but is only an example of the structural element disclosed in claims of the present invention, and the embodiment which is included in the technical spirit described in the entire specification and includes the structural elements which can be substituted with the equivalents in the claims may be included in the scope of the appended claims.

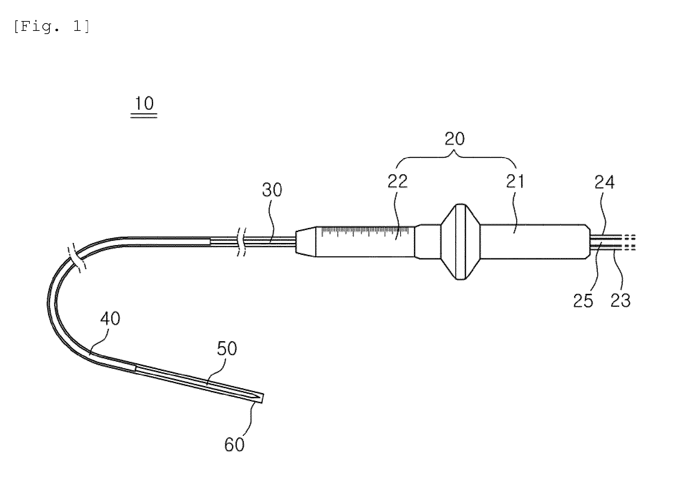

Fig. 1 is a perspective view of a high-frequency heat therapy electrode device equipped with a flexible tube according to one embodiment of the present invention;

As shown in Fig. 1, a high-frequency heat therapy electrode device 10 equipped with a flexible tube according to one embodiment of the present invention is a medical equipment provided with an electrode needle 50 disposed in front of a handle 20 and cauterizing a lesion site with the high-frequency heat generated from the electrode needle 50 to necrotize the site, and a bendable and deformable flexible tube 40 is provided between the handle 20 and the electrode needle 50.

Here, the handle 20 includes a gripping part 21 on which an operator grips and a sliding part 22 which can be slid into the gripping part 21.

In addition, a pushing rod 30 formed of metal material and coupled to one side of the gripping part 21 is protruded from a front end of the sliding part 22. A rear end of the flexible tube 40 is coupled to a front end of the pushing rod 30 and a rear end of the electrode needle 50 is coupled to a front end of the flexible tube 40.

At this time, the pushing rod 30 is the element on which the operator grips, and the operator adjusts an insertion direction and exerts a force though the pushing rod. The pushing rod moves the electrode needle 50 to the lesion site and transmits an insertion force to allow the electrode needle 50 to be inserted in the tissue. For example, the pushing rod is formed of material, such as stainless steel, having sufficient strength.

In addition, the flexible tube 40 receives the force from the pushing rod 30 to push the electrode rod 50, and can be bent and inserted along, for example, a working channel (not shown) of an endoscope.

At this time, the flexible tube 40 transmits the force transmitted from the pushing rod 30 to the electrode needle 50 so as to enable the electrode needle 50 to pierce and penetrate a lesion site.

Accordingly, it is preferable that the flexible tube 40 is formed of synthetic resin material, which has a prescribed hardness and can be bent and deformed, such as PEEK(Polyether Ether Ketone).

In addition, the electrode needle 50 is an element which radiates the high-frequency to coagulate and necrotize the surrounding tissue when inserting in the tissue of the lesion site, and it is preferable that the electrode needle is formed of metal material such as stainless steel, which is harmless to humans, does not rust and has conductivity.

At this time, a portion of the electrode needle 50 may be coated with insulation material, and this is because heat is not generated on the insulative portion to divide the tissue into a portion which is cauterized and a portion which is not cauterized for performing an operation according to an insertion depth when the electrode needle 50 is inserted in the tissue.

Here, a hollow part is formed in the pushing rod 30, the flexible tube 40 and the electrode needle 50, and a cooling water circulation line for cooling the electrode needle 50 and a sensor line 25 connected to a temperature-measuring sensor 51 for measuring a temperature of cooling water are provided in the hollow part.

As shown in Fig. 1, for this purpose, a cooling water supplying tube 23, a cooling water discharging tube 24 and the sensor line 25 are connected to a rear end of the gripping part 21, cooling water is supplied to the cooling water circulation line via the cooling water supplying tube 23, cooling water passed through the cooling water circulation line is discharged to an outside through the cooling water discharging tube 24, and the sensor line 25 is extended to a front end portion of the electrode needle 50 and connected to the temperature-measuring sensor 51.

At this time, the sensor line 25 consists of two dissimilar metal wires which are coated with insulation material, ends of the wires are electrically connected to each other by means of a soldering so that the ends of the wires act as the temperature-measuring sensor 51.

Meanwhile, the pushing rod 30, the flexible tube 40 and the electrode needle 50 are surrounded by a sheath tube 60 formed of material which is flexible and has prescribed hardness, and the sheath tube 60 is coupled to a front end portion of the sliding part 22 so that the sheath tube can be moved forward and rearward together with the sliding part 22.

In other words, once the sliding part 22 is moved rearward and then accommodated in the gripping part 21, the sheath tube 60 surrounding the electrode needle 50 is also moved rearward so that the electrode needle 50 surrounded by the sheath tube 60 is exposed to an outside and can be inserted into a tissue of the lesion site to perform the high-frequency heat therapy.

On the contrary, if the sliding part 22 is moved forward from the gripping part 21, the sheath tube 60 is moved forward and surrounds the exposed electrode needle 50, and the electrode needle 50 is received in the sheath tube 60.

Preferably, when a front end of the sliding part 22 is coupled to the working channel of the endoscope in a luer lock manner and a front end portion of the sheath tube 60 is placed at the lesion site, an operator pushes the gripping part 21 to expose the electrode needle 50 from the sheath tube 60 and to insert the electrode needle into the lesion site, and then performs the high-frequency heat therapy.

At this time, once the high-frequency heat therapy is completed, the operator pulls the gripping part 21 to accommodate the electrode needle 50 in the sheath tube 60 again.

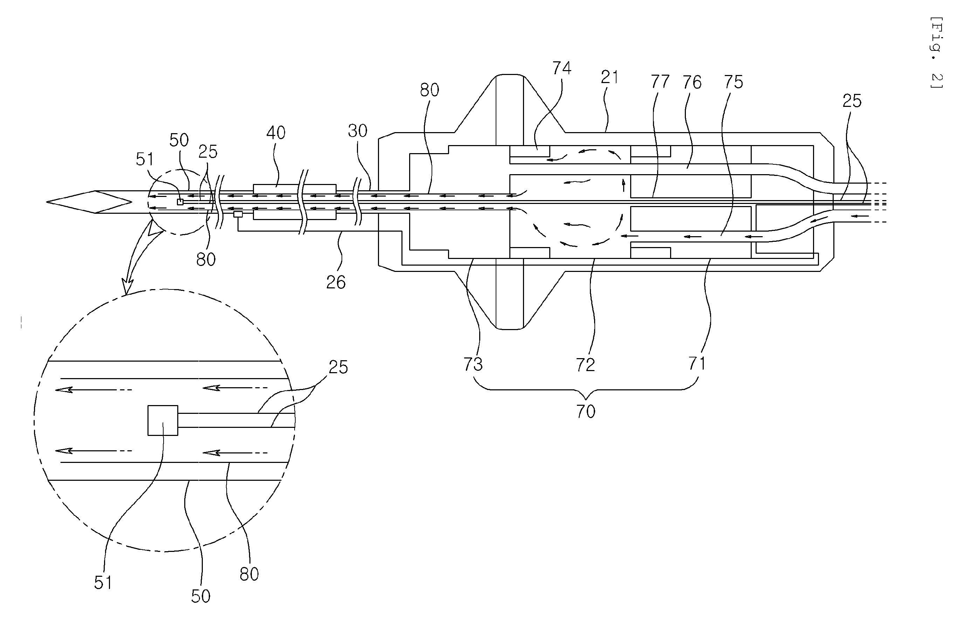

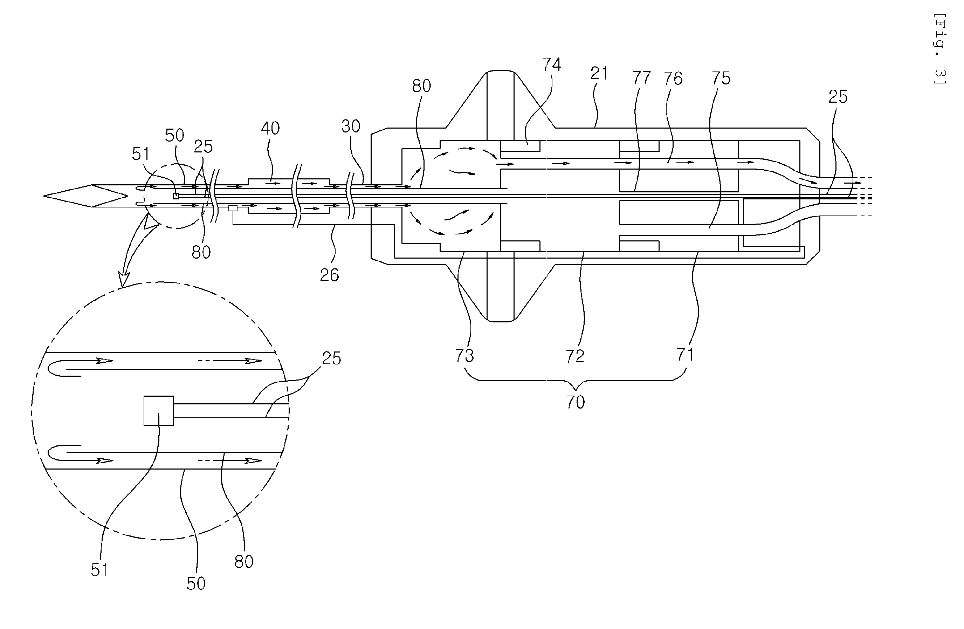

Fig. 2 is a schematic view showing a state in which cooling water is supplied to the electrode needle according to one embodiment of the present invention, and Fig. 3 is a schematic view showing a state in which cooling water is collected from the electrode needle according to one embodiment of the present invention. For easily understanding a circulation process of cooling water, apart from structures of the sheath tube 60 and the sliding part 22, the device is illustrated.

When the high-frequency heat therapy is performed, while the tissue is coagulated and necrotized by high-frequency frictional heat, moisture in the tissue is simultaneously vaporized by heat. As a result, a carbonization phenomenon in which the lesion site is stuck to an end of the electrode needle 50 is generated.

The carbonization of the electrode needle 50 causes a difficulty of high-frequency flow of the electrode needle 50 to make it difficult to cauterize the lesion site, and also causes a difficulty of a separation of the electrode needle 50 so that there is need to maximally suppress moisture vaporization from the tissue caused by heat.

For the above purpose, in the high-frequency heat therapy electrode device 10 equipped with the flexible tube according to one embodiment of the present invention, a cooling water circulation line in which cooling water is circulated is formed in the electrode needle 50. This structure is described in more detail below.

As shown in Fig. 2, a cooling water circulation block 70 is provided at one side of an interior of the gripping part 21, and this cooling water circulation block 70 divides a flow passage into each flow passage so as to prevent cooling water supplied to the electrode needle 50 and cooling water collected from the electrode needle 50 from being mixed with each other.

At this time, it is preferable that the cooling water circulation block 70 has a shape corresponding to that of the gripping part 21 to enable the cooling water circulation block to be accommodated in the gripping part 21, the cooling water circulation block may be formed integrally with the gripping part, and unit blocks of at least one or more synthetic resin material are assembled to form the cooling water circulation block 70.

For example, as shown in Fig. 2, a first block 71, a second block 72 and a third block 73, which have a cylindrical shape in general, may be sequentially coupled to each other by a method such as an ultraviolet bonding to form the cooling water circulation block 70, and it is preferable to fit a packing 74 at a coupling portion between the adjacent blocks for preventing cooling water from being leaked.

At this time, the fist block 71 is disposed at a rearmost end of the cooling water circulation block 70, and the cooling water supplying tube 23 for supplying cooling water, the cooling water discharging tube 24 for discharging cooling water and the sensor line 25 extended to a front end portion of the electrode needle 50 and connected to the temperature-measuring sensor 51 are connected to a rear end of the first block 71.

And, a cooling water supplying passage 75 and a cooling water discharging passage 76 spaced apart from each other are formed in the first block 71, and a sensor guiding line 77 is formed between the passages.

At this time, the cooling water supplying passage 75 is connected to the cooling water supplying tube 23, the cooling water discharging passage 76 is connected to the cooling water discharging tube 24, and the sensor line 25 traverse the first block 71 through the sensor guiding line 77. It is preferable to secure one side of the sensor line 25 to one side of an inner wall of the sensor guiding line 77 by means of a method such as a soldering.

The second block 72 is provided in front of the first block 71, and the sensor line 25 is extended from the first block 71 and transverses the second block 72.

In addition, a space is formed in the second block 72, and the cooling water supplying passage 75 of the first block 71 is communicated with this space. Therefore, cooling water supplied through the cooling water supplying passage 75 flows into the internal space of the second block 72.

At this time, the cooling water discharging passage 76 is extended from the first block 71 and transverses the second block 72 so that unused cooling water guided in the second block 72 along the cooling water supplying passage 75 and used cooling water flowing through the cooling water discharging passage 76 are not mixed with each other, but flow in a separated state.

The third block 73 is provided in front of the second block 72, a space is formed in the third block 73, and the cooling water discharging passage 76 of the second block 72 is communicated with this space. Therefore, used cooling water flowing into the third block 73 is discharged through the cooling water discharging passage 76.

In addition, a guide tube 80 in which the sensor line 25 is accommodated transverses the third block 73, the guide tube is coupled such that a rear end of the guide tube 80 is communicated with the space of the second block 72, and a front end of the guide tube 80 passes through the pushing rod 30 and the flexible tube 40 and is then extended to a front end portion of the electrode needle 50.

Therefore, unused cooling water of the second block 72 is supplied to a front end portion of the electrode needle 50 through the guide tube 80.

Arrows shown in Fig. 2 indicate a flow of unused cooling water, and a process for supplying unused cooling water is described again with reference to Fig. 2 as below.

First of all, unused cooling water supplied through the cooling water supplying tube 23 passes through the cooling water supplying passage 75 of the first block 71 and flows into the second block 72.

And, as the guide tube 80 is extended from the second block 72 to a front end portion of the electrode needle 50, unused cooling water of the second block 72 is supplied to a front end portion of the electrode needle 50 through the guide tube 80.

Meanwhile, arrows shown in Fig. 3 indicate a flow of used cooling water, and a process for collecting used cooling water is described again with reference to Fig. 3 as below.

Cooling water supplied into the electrode needle 50 through the guide tube 80 cools a front end portion of the electrode needle 50 and is then collected, and used cooling water collected through the electrode needle 50, the flexible tube 40 and the pushing rod 30 flows a gap formed between an outer circumference surface of the guide tube 80 and each inner circumference surfaces of the electrode needle 50, the flexible tube 40 and the pushing rod 30.

And, since the pushing rod is coupled such that a rear end of the pushing rod 30 is communicated with the space of the third block 73, used cooling water passing through the electrode needle 50, the flexible tub 40 and the pushing rod 30 flows into the third block 73, passes through the second block 72 and the first block 71 and is collected in the cooling water discharging tube 24 through the cooling water discharging passage 76 communicated with the space of the third block 73, and is finally discharged to an outside.

At this time, a flow of cooling water can be achieved, for example, by means of a pump (not shown) separately provided at an outside, and it is also possible to lower a temperature of collected cooling water and resupply cooling water to the electrode needle 50 through the cooling water supplying tube 23.

Meanwhile, a temperature-measuring sensor 51 is provided in a front end portion of the guide tube 80.

This temperature-measuring sensor 51 is provided for checking a temperature of cooling water, and a sensor line 25 is connected to the temperature-measuring sensor 51.

At this time, the sensor line 25 passes sequentially through the sensor line-guiding tube 77 of the first block 71, a space part of the second block 72 and the guide tube 80 and then is connected to the temperature-measuring sensor 51.

In addition, in order to transmit high-frequency outputted from a high-frequency oscillator (not shown) which is separately provided, to the electrode needle 50, an electrode line 26 is connected to one side of the electrode needle 50, a wire coated with insulation material may be employed as this electrode line 26, and this electrode line 26 is extended from the high-frequency oscillator to a rear end of the gripping part 21.

At this time, the electrode line 26 is extended into the sheath tube 60 along an outer circumference surface of the cooling water circulation block 70, this electrode line 26 is extended through a gap formed between an inner circumference surface of the sheath tube 60 and an outer circumference surface of each of the pushing rod 30, the flexible tube 40 and the electrode needle 50, and a front end of the electrode line 26 is connected to one side of an outer circumference surface of the electrode needle 50.

According to one embodiment of the present invention, as shown in Fig. 2 and Fig. 3, after the electrode line 26 is inserted into the first block 71, it is bent and escaped from a rear end of the first block 71, and is extended in close contact with outer circumference surfaces of the cooling water circulation block 70, the pushing rod 30 and the flexible tube 40. Then, the electrode line may be connected to one side of an outer circumference surface of a front end portion of the electrode needle 50. The above connection structure of the electrode line 26 may be modified diversely as needed.

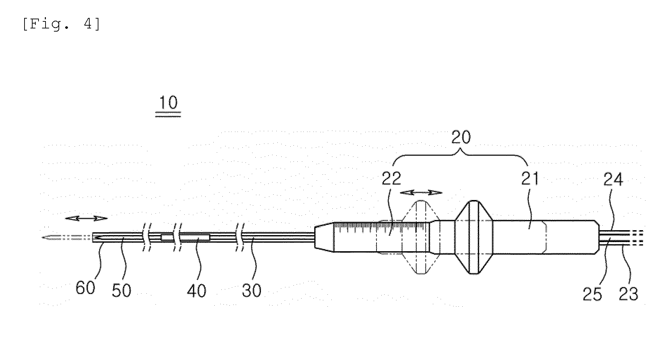

Fig. 4 is a view showing a state in which the high-frequency heat therapy electrode device equipped with the flexible tube according to one embodiment of the present invention is in use, more particularly, showing the device when the high-frequency heat therapy is performed using the working channel of the endoscope.

An operation of the high-frequency heat therapy electrode device 10 quipped with the flexible tube according to one embodiment of the present invention is carried out as below.

If the high-frequency heat therapy is performed using the endoscope, an operator inserts the electrode needle 50 into the working channel of the endoscope and pushes the flexible tube 40 with the pushing rod 30 to allow the electrode needle 50 to reach the lesion site.

At this time, the pushing rod 30, the flexible tube 40 and the electrode needle 50 are in a state in which they are accommodated in the sheath tube 60, and a front end portion of the sliding part 22 is secured to the working channel of the endoscope in the luer lock manner.

Since the flexible tube 40 has a prescribed hardness and can be bent, the electrode needle 50 is easily moved to the lesion site along a curved shape of tubular organ in human body, for example, such as coelom or large intestine, and can be inserted into the tissue by an insertion force transmitted through the flexible tube 40.

In addition, since the electrode needle 50 is accommodated in the sheath tube 60 while transferring, a damage of endoscope equipment or human tissue caused by a tip of the electrode needle 50 is prevented.

When the electrode needle 50 reaches the lesion site, as shown in Fig. 4, the operator grips and pushes the gripping part 21 of the handle 20 to allow a front end of the electrode needle 50 to be protruded from the sheath tube 60 and be inserted into the tissue, and then performs the high-frequency heat therapy using the high-frequency output transmitted from the high-frequency oscillator to the electrode needle 50 through the electrode line 26.

At this time, in order to prevent the carbonization of the electrode needle 50 from being generated, cooling water is supplied and circulated into the electrode needle 50. If cooling water is supplied through the cooling water supplying tube 23 connected to a rear end of the handle 20, cooling water is supplied to the second block 72 via the cooling water supplying passage 75 of the first block 71 provided in the handle 20.

Then, cooling water is supplied up to a front end portion of the electrode needle 50 through the guide tube 80 extended from the second block 72 to a front end portion of the electrode needle 50.

Used cooling water passes sequentially through a gap between an inner circumference surface of the electrode needle 50 and an outer circumference surface of the guide tube 80, a gap between an inner circumference surface of the flexible tube 40 and an outer circumference surface of the guide tube 80 and a gap between an inner circumference surface of the push rod 30 and an outer circumference surface of the guide tube 80, and is returned to the third block 73. Then, cooling water passes sequentially through the second block 72 and the first block 71 via the cooling water discharging passage 76 and is discharged through the cooling water discharging tube 24. It is possible to lower a temperature of cooling water collected as described above and to resupply it to the cooling water supplying tube 23.

At this time, it is possible to check a temperature of cooling water through the temperature-measuring sensor 51 placed at a front end portion of the electrode needle 50.

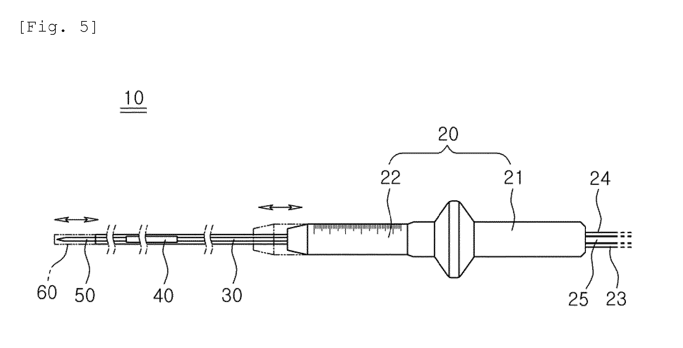

Fig. 5 is a view showing a state in which a high-frequency heat therapy electrode device equipped with a flexible tube according to another embodiment of the present invention is in use, more particularly, showing the device when the high-frequency heat therapy is performed without using the endoscope.

As shown in Fig. 5, according to another embodiment of the present invention, the operator pulls the sliding part 22 of the handle 20 to expose the electrode needle 50 from the sheath tube 60, inserts the electrode needle 50 into the tissue of the lesion site and then performs the high-frequency heat therapy for the tissue of the lesion site using the high-frequency output transmitted from the high-frequency oscillator to the electrode needle 50 through the electrode line 26.

### [Industrial Applicability]

According to the high-frequency heat therapy electrode device equipped with the flexible tube in accordance with one embodiment of the present invention, since the flexible tube having a prescribed hardness is provided at a rear end of the electrode needle, the flexible tube can be bent and inserted up to the lesion site and the insertion force for sticking the electrode needle in the tissue of the lesion site is sufficiently transmitted so that the flexible tube is inserted into the working channel of the endoscope to enable an effective high-frequency heat therapy using the endoscope to be performed.

In addition, since the cooling water circulation line is formed in the electrode needle to suppress moisture vaporization from the tissue of the lesion site and to prevent a generation of the carbonization of the electrode needle, the effective high-frequency heat therapy can be performed and it is possible to check a supplying state, such as a temperature of cooling water, through the temperature-measuring sensor provided in the electrode needle.

At this time, since the flexible tube has a prescribed hardness, due to a bending of the flexible tube, it is possible to prevent the cooling water circulation line and the sensor line in the electrode needle from being folded and closed or broken.

In addition, when the device is stored and transferred, or inserted in the working channel of the endoscope, the electrode needle is accommodated in the sheath tube, and the electrode needle can be exposed to an outside only when needed such as the high-frequency heat therapy so that unexpected damage of the equipment or the human tissue can be prevented in advance.

## Claims

1. A high-frequency heat therapy electrode device provided with an electrode needle disposed in front of a handle and cauterizing a lesion site with the high-frequency heat generated from the electrode needle to necrotize the lesion site, comprising a flexible tube provided between the handle and the electrode needle and being easily bent and deformed.

2. The high-frequency heat therapy electrode device of claim 1, wherein the flexible tube has a prescribed hardness to enable the electrode needle to be inserted into the lesion site along a working channel of an endoscope by means of a movement of the handle.

3. The high-frequency heat therapy electrode device of claim 1, wherein the electrode needle is accommodated in a sheath tube and a front end portion of the electrode needle is exposed out of the sheath tube when the device is used.

4. The high-frequency heat therapy electrode device of claim 3, wherein the sheath tube is provided with an electrode line received therein, and a front end of the electrode line is connected to one side of an outer surface of the electrode needle.

5. The high-frequency heat therapy electrode device of claim 3, wherein the handle comprises a gripping part and a sliding part which can be slid into the gripping part, and the sheath tube can be moved together with the sliding part.

6. The high-frequency heat therapy electrode device of claim 1, comprising a cooling water circulation block provided at one side of an interior of the handle for supplying and circulating cooling water into the electrode needle.

7. The high-frequency heat therapy electrode device of claim 6, wherein the cooling water circulation block comprises a first block to which a cooling water supplying tube and a cooling water discharging tube are connected, the first block having a cooling water supplying passage and a cooling water discharging passage formed therein; a second block to which the cooling water discharging passage is extended, the second block being coupled to one side of the first block and communicated with the cooling water supplying passage; and a third block coupled to one side of the second block and communicated with the cooling water discharging passage.

8. The high-frequency heat therapy electrode device of claim 6, wherein one side of the cooling water circulation block is coupled to a guide tube extended into the electrode needle for supplying cooling water.

9. The high-frequency heat therapy electrode device of claim 8, comprising a temperature-measuring sensor provided at one side of an interior of the guide tube.

10. The high-frequency heat therapy electrode device of claim 1, comprising a pushing rod provided between the flexible tube and the handle.
